# EUROPEAN PATENT APPLICATION

(11) **EP 4 789 636 A1**
(43) Date of publication of application: **12.08.2026**
(21) Application number: 25305183.3
(22) Date of filing: 07.02.2025
(51) Int. Cl.: A61B 34/20, A61F 2/40

(54) **MULTIFUNCTIONAL SURGICAL INSTRUMENT FOR USE IN COMPUTER-ASSISTED SHOULDER ARTHROPLASTY**

(71) Applicant: SCAP Hologram, 38400 Saint-Martin-D'Hères (FR)
(72) Inventor: CARDON, Jean-Emmanuel, 38400 SAINT-MARTIN-D'HÈRES (FR); HENRY, Delphine, 38400 SAINT-MARTIN-D'HÈRES (FR); SCHERS, Jonathan, 38400 SAINT-MARTIN-D'HÈRES (FR)
(74) Representative: Regimbeau

(57) **Abstract**

The invention concerns a multifunctional surgical instrument (1) designed for use in computer-assisted shoulder arthroplasty, comprising
- a handle (11) adapted to be manipulated by an operator, the handle (11) comprising an elongated body extending along a longitudinal axis X, the elongated body comprising a proximal end (12) and a distal end (13);
- an extension pole (14) attached to the handle (11) at the proximal end (12) of the handle (11), the extension pole (14) extending from the proximal end (12) of the handle (11),
- at least one stem (2) comprising an operating tip (21, 31, 41) intended to be in contact with at least one bone, each operating tip (21) being designed to perform a specific surgical function and being adapted to be connected to the stem (2);
the extension pole (14) being adapted to rigidly attach an EM instrument receiver (R1) of an EM localization system (LOC) at a known location relative to the distal end (13) of the handle (11), enabling real-time spatial tracking of the distal end (13) of the handle (11) and, consequently the operating tip (21).

## Description

### FIELD OF THE INVENTION

The invention relates to surgical instruments used in computer-assisted shoulder arthroplasty and more specifically to a multifunctional surgical instrument equipped with multiple tips, each corresponding to a specific for the surgery.

### BACKGROUND OF THE INVENTION

Surgery of a bone and in particular shoulder replacement surgery is a complicated type of surgery. Shoulder replacement surgery is becoming increasingly common because of its ability to alleviate pain and restore range of motion in many patients. Shoulder arthroplasty (anatomic and reversed) has been demonstrated to successfully recover function to shoulders affected by arthrosis and rotator cuff insufficiency.

Performing such a procedure requires several instruments, including a probe, a drill guide, and a reamer. These instruments are typically reusable. The surgical procedure begins with the use of the probe, followed by the drill guide, then the reamer. The probe is then used again to verify the reaming.

In computer-assisted surgery, these instruments must be tracked and located in real time. Typically, a single tracking sensor is used and must be transferred from one instrument to another during the procedure. This repeated handling increases the risk of mispositioning and results in a loss of time.

### BRIEF DESCRIPTION OF THE INVENTION

The invention aims at limiting the number of steps required for using different instruments during shoulder arthroplasty.

According to a first aspect, the invention concerns a Multifunctional surgical instrument designed for use in computer-assisted shoulder arthroplasty, comprising
- a handle adapted to be manipulated by an operator, the handle comprising an elongated body extending along a longitudinal axis X, the elongated body comprising a proximal end and a distal end;
- an extension pole attached to the handle at the proximal end of the handle, the extension pole extending from the proximal end of the handle,
- at least one stem comprising an operating tip intended to be in contact with at least one bone, each operating tip being designed to perform a specific surgical function and being adapted to be connected to the stem;
the extension pole being adapted to rigidly attach an EM instrument receiver of an EM localization system at a known location relative to the distal end of the handle, enabling real-time spatial tracking of the distal end of the handle and, consequently the operating tip.

The instrument according to the first aspect comprises one or more of the following features alone in combination:
- The elongated body is hollow, and the stem is adapted to be inserted and secured into the elongated body.
- The stem has a known length defined along the longitudinal axis so that the tracking of the distal end allows the tracking of the operating tip.
- The extension pole comprises a rigid arm extending from the elongated body, a platform for supporting an EM instrument receiver extending from the rigid arm, the platform allowing the EM instrument receiver to be as close as possible to the distal end without disturbing the manipulation of the instrument.
- Each operating tip is selected from the group consisting of: a registration probe configured to designate a bone surface for registration purpose; a drill guide configured to receive a pin for guiding a reamer or a drill bit for preparing a bone to receive a component;
a reamer configured for conforming a bone to receive an implant.

According to a second aspect of, the invention concerns a system for calibrating the instrument of the first aspect, comprising
- a calibration unit, comprising:
   - a plate comprising a support for reproductible attachment of an EM transmitter to the plate,
   - at least one receiving site arranged on the plate, the receiving site being adapted for receiving the handle of the multifunctional surgical instrument so that the distal end of the handle rests on the plate at a calibrating position, the plate and the receiving site having a known geometry;
- an EM localization system comprising: an EM transmitter arranged on the support on the plate; an EM instrument receiver attached to the extension pole,
- a processing unit configured for implementing a step of calibrating the EM instrument receiver relative to the distal end of the instrument allowing to locate the distal end of the instrument when the EM instrument receiver receives EM signals from the EM transmitter.

The system according to the second aspect comprises one or more of the following features alone in combination:
- The step of calibrating the EM instrument receiver comprises: processing EM signals received by the EM instrument receiver, while the EM transmitter emits EM signals and the instrument is rotated, in order to determine geometrical transformations of the EM transmitter relative to the EM instrument receiver; determining, from these geometrical transformations, the trajectory described by the EM instrument receiver, said trajectory forming a circular arc; identifying the center of the circular arc within the coordinate system of the EM transmitter; determining the longitudinal axis of the instrument as the normal to the plane containing the trajectory points and passing through the center of the circular arc; determining the position of the distal end of the instrument by computing the intersection between the plane of the plate and the longitudinal axis of the instrument.
- The EM instrument receiver and the EM bone receiver are intended to be located relative to each other at a distance between 5 and 15 cm, preferably 10 cm.

The plate is configured to be non-magnetic-field-disturbing by material and/or by design.

A non-magnetic-field-disturbing material is plastic or titanium.

### BRIEF DESCRIPTION OF THE DRAWINGS

Further features and advantages of the invention will be apparent from the description that follows, based on the appended drawings, wherein:
- Figure 1 illustrates a surgical field where the invention is implemented.
- Figure 2 illustrates a handle of a multifunctional instrument of the invention.
- Figure 3 illustrates a multifunctional instrument according to one embodiment of the invention in registration probe configuration.
- Figure 4 illustrates a multifunctional instrument according to one embodiment of the invention in drill guide configuration.
- Figure 5 illustrates a multifunctional instrument according to one embodiment of the invention in reamer configuration.
- Figure 6, figure 7 and figure 8 illustrate a calibration unit for calibrating the instrument of the invention.

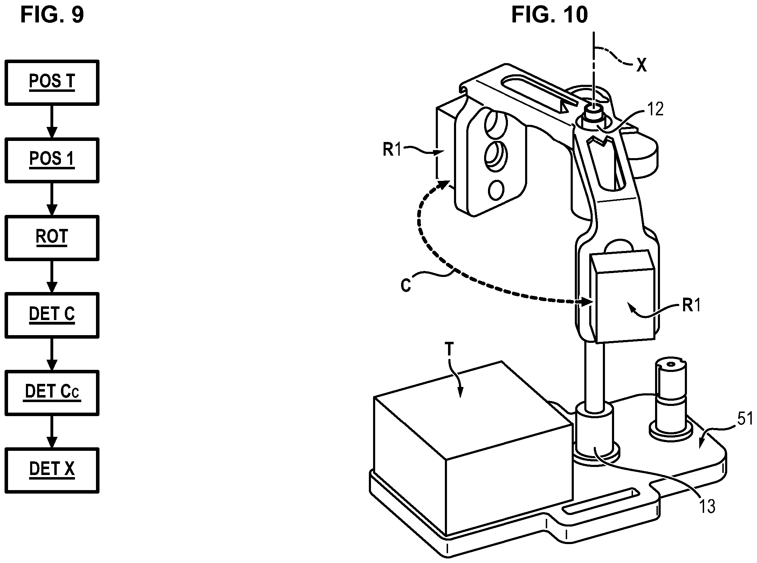

- Figure 9 and figure 10 illustrate the calibrating process of the instrument of the invention.
- Figure 11 illustrates the calibration error between two configurations.

On the figures, similar elements have identical references.

### DETAILED DESCRIPTION OF EMBODIMENTS OF THE INVENTION

*Presentation* of a *surgical field where performing shoulder arthroplasty is performed*

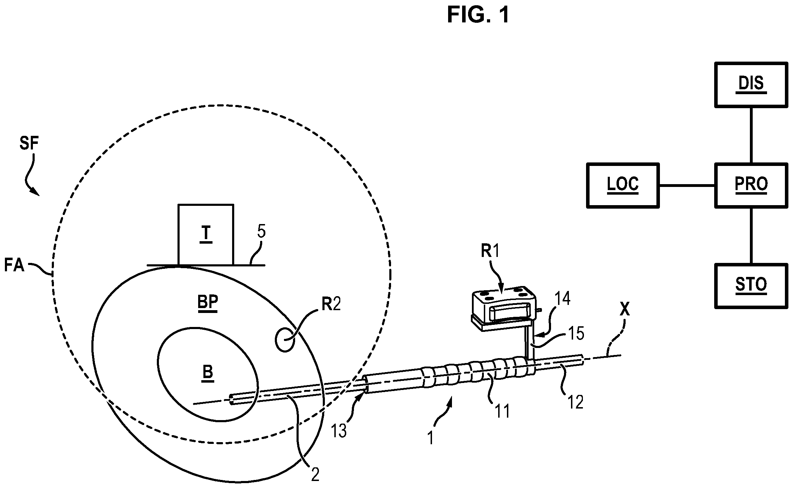

**Figure 1** shows a surgical field SF in which the invention is implemented during a shoulder surgery, specifically a shoulder arthroplasty. During this procedure, an implant (not shown) is positioned within a bone surface B. For instance, a glenoidal implant or a humeral implant is positioned on the glenoid cavity or on the humerus.

The positioning of this implant requires correct access to bone surface B. Thus, tissues around bone surface B are preferably retracted by means of a retractor assembly (not shown) comprising retractors allowing access to bone surface B during the surgery.

During the surgery, an operator uses a multifunctional surgical instrument 1.

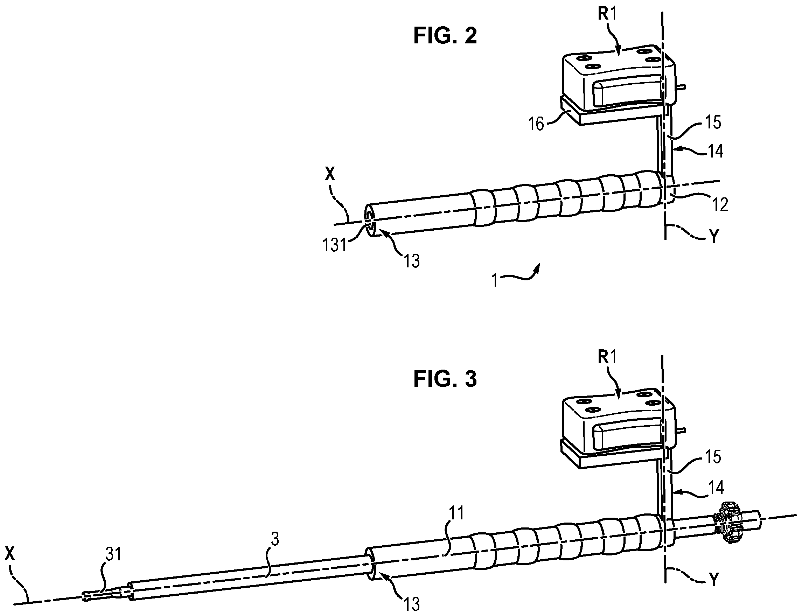

As illustrated on figure 2, this instrument 1 comprises a handle 11 adapted to be manipulated by the operator, this handle 11 comprises an elongated body extending along a longitudinal axis X, the elongated body comprising a proximal 12 end and a distal end 13.

In relation to figure 3, figure 4 and figure 5, at the distal end 13, a stem 2, 3, 4 with an operating tip 21, 31, 41 is attached. The operating tip 21, 31, 41 is intended to be in contact with the bone surface B and designed to perform a specific surgical function.

The multifunctional surgical instrument 1 needs to be tracked during the surgery. To this end, a system S is provided and comprises a processing unit PRO connected to a localization system LOC. A storage unit STO and a display unit DIS are connected to the processing unit PROC for storing data acquired and for displaying images useful for the surgery.

### Localization system

The localization system LOC comprises an EM transmitter T and at least two EM receivers R1, R2. By processing the transmitted and received signals, the processing unit PRO permits to locate each EM receiver relative to each other and thus permits to locate the instrument 1. The EM transmitter T, the EM receivers R1, R2 are positioned within a functional area FA, defined by the range of the EM transmitter T: an EM instrument receiver R1 is rigidly attached to the multifunctional surgical instrument 1 and a EM bone receiver R2 is attached to a body part BP. The EM transmitter T is placed within the surgery field SF and defines the functional area FA.

The EM bone receiver R2 plays a crucial role, as it serves as a reference point for locating the EM instrument receiver R1 during surgery. Since EM bone receiver R2 remains stationary throughout the procedure, it ensures accurate tracking of the instrument 1.

To maintain stability, the EM bone receiver R2 is positioned to withstand vibrations from the instrument 1 and other disturbances occurring during surgery. For example, EM bone receiver R2 can be rigidly attached to the scapula. More generally, it is secured to a reference structure that remains fixed and unaltered throughout the procedure.

During the surgery, the EM transmitter T and the EM receiver R1, R2 are positioned within the surgical field SF within the functional area FA defined by the range of the EM transmitter T (represented by the circle on figure 1). Indeed, the signal of the EM transmitter T decreases as a function of d³, d being the distance between the EM transmitter T and an EM receiver R2. Therefore, it is essential to position the EM receivers R1, R2 relative to the EM transmitter T so that the electromagnetic signals are sufficient to ensure accurate localization. The EM transmitter T is preferably attached to the patient itself or more generally as close as possible to the region to be accessed while avoiding interference with the operator during the surgery.

The EM transmitter T is preferably supported by a support 5. As detailed further, according to one embodiment, this support 5 can also function as a calibration unit CAL for the instrument 1.

Alternatively, the EM transmitter T can be supported by any structure in functional area FA.

For the surgery, and to ensure accurate localization, the EM transmitter T and the EM receiver R1, R2 are positioned within the functional area FA so that
(i) each EM receivers R1, R2 is within a range of 5 to 15 centimeters, preferably 10 centimeters from the EM transmitter T; and
(ii) magnetic-field-disturbing object(s) are avoided between the EM transmitter T and the EM receivers R1, R2.

The goal is to define in the surgical field SF perturbing emitting area(s), i.e., that is/are susceptible to perturb the localization system LOC based on EM waves. A perturbing area is for instance around one or more element(s) susceptible to disturbing the localization system LOC.

Using an EM localization system LOC offers significant advantages, as it enhances access, precision, and navigation during procedures compared to other systems, such as optical localization systems.

### Multifunctional surgical instrument

As already introduced, the multifunctional surgical instrument 1 comprises a handle 11 comprising an elongated body extending along a longitudinal axis X, the elongated body comprising a proximal 12 end and a distal end 13 (see figure 2).

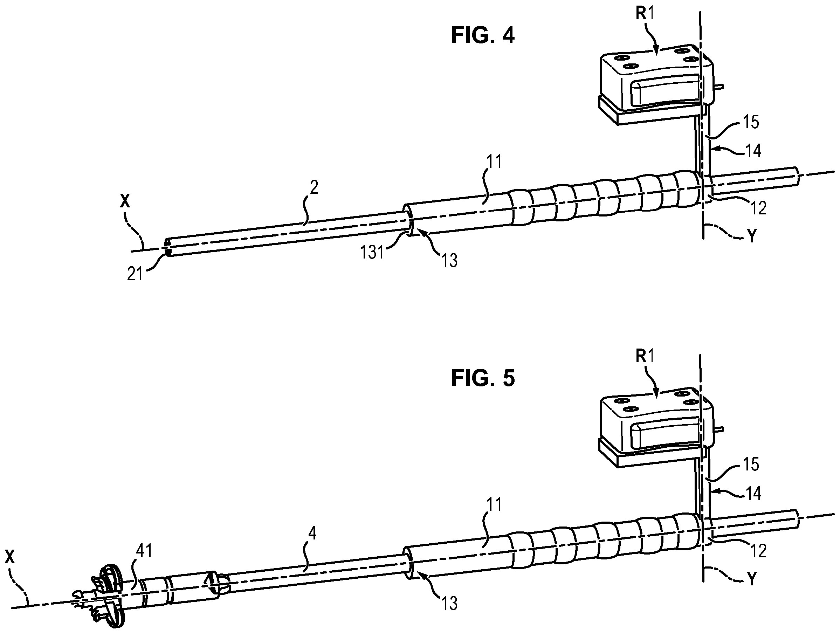

This handle is the same for several surgical functions. To this end, at the distal end 13, a stem 2, 3, 4 is attached to the handle 11. The stem 2, 3, 4 comprises an operating tip 21, 31, 41 selected from the group consisting of:
- a registration probe 31 configured to designate a bone surface for registration purpose (see figure 3);
- a drill guide 21 configured to receive a pin for guiding a reamer or a drill bit for preparing a bone to receive a component (see figure 4);
- a reamer 41 configured for conforming a bone to receive an implant (see figure 5).

The stem 2, 3, 4 is detachable allowing calibration of the handle 11 (see below).

The stem 2, 3, 4 is attached to the handle 11 by resting on the distal end for instance by compression or by support using a thread or elastic flange.

According to one embodiment, for each surgical function, one stem corresponds to one operating tip. In that case, for switching between the surgical function, the operator needs to replace the stem with the corresponding tip.

According to one embodiment, for the drill guide and for the registration probe, the stem 2, 3 is the same. In that case, the stem 2, 3 belongs to the drill guide. The drill guide 21 comprises the stem 2 which is a hollow tube having teeth at its end intended to be in contact with the bone. The drill guide permits to put a pin on the bone serving as a guide for the reamer. The drill guide being hollow, then it can be used for assembling the registration probe into the drill guide (see figure 3). To do that, the operator only needs to insert the registration probe through the proximal end of the elongated body 11 (see figure 3) and through the drill guide. For the reamer, the operator uses a specific stem 4 with a corresponding operating tip 41.

The instrument 1 covers typical tools that can be used during shoulder arthroplasty.

Preferably, the elongated body is hollow and comprises an opening 131 at its distal end 13, into which the stem 2, 3, 4 is inserted.

The handle is preferably configured to be non-magnetic-field-disturbing (by its material and/or design). A non-magnetic-field-disturbing material is for instance plastic or titanium. A non-magnetic-field-disturbing design means that this property can be obtained with the design as such.

The operating tip is preferably metallic or is in plastic or is in ceramic.

For attaching the EM instrument receiver R1, an extension pole 14 is attached to the handle at the proximal end of the handle, the extension pole 14 extending from the proximal end of the handle 11.

The extension pole 14 can be attached to the handle 11 in multiple ways. The main constraint is that the extension pole 14 must be fixed and immobile relative to the handle 11. The extension pole 14 is preferably integrated as a single piece with the handle 11 and can be manufactured, for instance using 3D printing.

Preferably, the extension pole 14 includes a rigid arm 15 that is elongated and extends along a longitudinal axis Y essentially perpendicularly to the longitudinal axis X. A platform 16 extends from the rigid arm 15 positioned at a distance and for instance parallel to the handle 11, in the direction of the distal end 13. The platform 16 supports the EM instrument receiver R1 that is positioned above the handle 11. The platform 16 comprises fixing elements (not shown) to ensure reproductible positioning of the EM instrument receiver R1.

Thus, the instrument 1 comprises a main piece consisting of the handle and the extension pole wherein the EM instrument receiver R1 is attached. Therefore, for using different surgical function, the operator only needs to use different stems without removing the EM instrument receiver R1.

Main advantage of this solution is that the error due to the assembly/disassembly mechanism is located near the distal end of the handle. Since deviation is afunction of the instrument length, having this assembly close to this distal end reduces this error.

There is also a cost-saving benefit, as only one handle is needed for all instruments tips.

From a usability perspective, it is easier for the operator to keep the handle connected to the EM receiver R1 in one hand and change the operating tip as needed, rather than replacing the entire instrument. The use of an extension pole 14 permits to keep the EM instrument receiver R1, away from the operating tip of the corresponding stem. The idea is to have the EM instrument receiver R1 as close as possible from the EM transmitter T1 during the surgery without obstructing its manipulation

Indeed, the instrument comprises materials (for instance metal) that can interfere with the EM field.

Extension pole 14 is configured to be non-magnetic-field-disturbing (by its material and/or design). The extension pole 14 has a known geometry.

The instrument 1 is very advantageous. During surgery, the operator only needs to change the operating tip to switch from one surgical function to another.

The EM receiver of the instrument needs to be calibrated relative to the EM transmitter T for accurate tracking during the surgery (see below for the calibration process).

With the instrument of the invention, a single calibration of the distal end of the housing is sufficient to track the operating tip throughout the surgery. Indeed, calibrating the instrument relative to this distal end is enough to ensure continuous tracking of the instrument, as the operating tip adds a negligible length compared to the length of the stem.

This eliminates the need for recalibration when the operator needs to switch from a function to another function or when usually the operator switches from an instrument to another instrument. Indeed, usually, when switching instruments, the receiver also needs to be repositioned, requiring a new calibration.

In conventional systems, no calibration is performed, or calibration is required each time an instrument is changed. With the invention's instrument, only one calibration is needed for the entire procedure. Calibrating the distal end of the handle represents a compromise between an acceptable error margin for navigation, which saves time during surgery, and allowing the operator to stay focused while maintaining greater precision in navigation compared to performing the procedure without calibration.

### Calibration unit

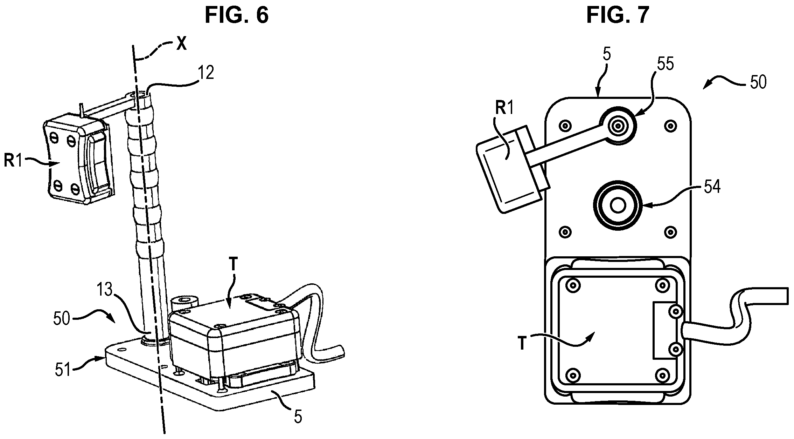

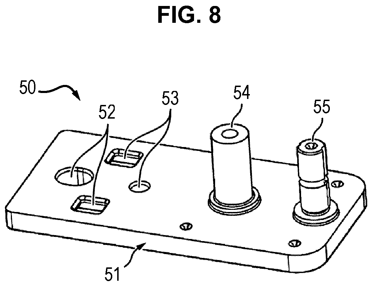

For calibrating the instrument 1, in relation with figure 6, figure 7, figure 8, a calibration unit 50 is provided. This calibration unit comprises a plate 51 comprising a support 5 for reproductible attachment of an EM transmitter to the plate. This support 5 comprises fixing components 52, 53. As illustrated, the fixing components 52, 53 are female imprints adapted for receiving complementary male imprints located on the EM transmitter T to be supported. The personskilled in the art will understand that any type of fixing element can be used.

The EM transmitter T can be attached to the support 5 for the entire duration of the surgery and thus not just only for calibrating purposes.

At least one receiving site 54, 55 is arranged on the plate 51, the receiving site 54, 55 being adapted for receiving the handle 11 of the multifunctional surgical instrument 1 so that the distal end 13 of the handle rests on the plate at a calibrating position, the plate and the receiving site having a known geometry (see figure 6).

Receiving site can be any shape complementary to the handle of the instrument 1, for example adjusted cylinder, divot, fins, depending on the type of desired check. It allows the EM instrument receiver R1 to be calibrated relative to the EM transmitter T.

The plate 51 is preferably configured to be non-magnetic-field-disturbing by material and/or design. In other words, it is designed so that it does not interfere with the magnetic field.

The calibration unit can be used for implementing calibration before the surgery. Also, during the surgery, the calibration unit enables verification of the correct calibration of the instrument 1.

The calibration unit can be used in conjunction with the EM localization system LOC and the processing unit PROC to calibrate the EM instrument receiver relative to the distal end of the instrument enabling precise location tracking of the distal end of the instrument when the EM instrument receiver receives EM signals from the EM transmitter.

The calibration of the instrument 1 is implemented as follows.

The EM transmitter T is positioned on the support 5 (step POS T).

The handle 11 of the instrument 1 is placed on the plate 51 such that the distal end 13 of the handle 11 rests on the plate 51 at a calibrating position (step POS 1).

The EM transmitter 1 emits EM signals, and the handle 11 is rotated around its longitudinal axis X (step ROT).

The EM instrument receiver R1 receives the EM signals during the rotation and the received signals are processed to determine the trajectory described by the EM instrument receiver, said trajectory forming a circular arc C (step DET C).

The center of the circular arc within the coordinate system of the EM transmitter T is determined (step DET Cc) and the coordinates of the longitudinal axis X are determined (step DET X) as being the normal to the plane containing the trajectory points and passing through the center of the circular arc C.

Then the position of the distal end 13 of the instrument 1 is determined (step DET 13) by computing the intersection between the plane of the plate and the longitudinal axis X of the instrument 1.

Finally, the EM instrument receiver R1 is calibrated relative to the distal end 13 of the instrument, allowing accurate location of the distal end 13 of the instrument when the EM instrument receiver receives EM signals from the EM transmitter.

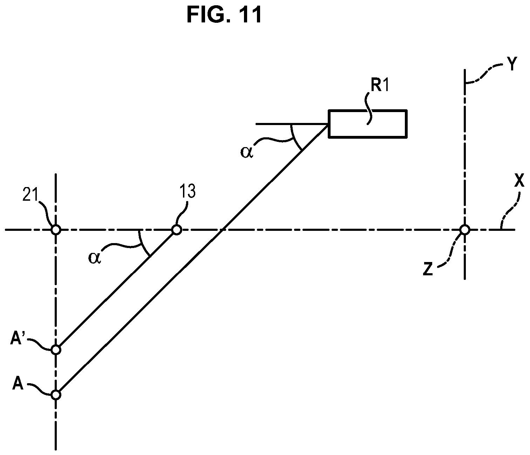

Figure 11 shows the error with the calibration of distal end 13 relative to the EM instrument receiver R1. Indeed, as can be seen on this figure 11, the error on the location of the operating tip 21 21 is smaller when the operating tip is located in relation to the distal end 13: location A is the location of the operating tip 21 when the EM instrument receiver is calibrated relative to the operating tip while A' is the location of the operating tip 21 when the EM instrument receiver is calibrated relative to the distal end 13.

In other words, the only degree of freedom in operating tip/stem assembly is along the X axis, so no Y or Z errors are introduced during assembly. Y and Z errors in EM instrument receiver positioning are set to zero by calibration and remain at zero during operating tip changes.

## Claims

1. A Multifunctional surgical instrument (1) designed for use in computer-assisted shoulder arthroplasty, comprising
- a handle (11) adapted to be manipulated by an operator, the handle (11) comprising an elongated body extending along a longitudinal axis X, the elongated body comprising a proximal end (12) and a distal end (13);
- an extension pole (14) attached to the handle (11) at the proximal end (12) of the handle (11), the extension pole (14) extending from the proximal end (12) of the handle (11),
- at least one stem (2, 3, 4) comprising an operating tip (21, 31, 41) intended to be in contact with at least one bone, each operating tip (21, 31, 41) being designed to perform a specific surgical function and being adapted to be connected to the stem (2, 3, 4);
the extension pole (14) being adapted to rigidly attach an EM instrument receiver (R1) of an EM localization system (LOC) at a known location relative to the distal end (13) of the handle (11), enabling real-time spatial tracking of the distal end (13) of the handle (11) and, consequently the operating tip (21, 31, 41).

2. The instrument of claim 1, wherein the elongated body is hollow, and the stem (2, 3, 4) is adapted to be inserted and secured into the elongated body.

3. The instrument of claims 1 to 2, wherein the stem (2, 3, 4) has a known length defined along the longitudinal axis (X) so that the tracking of the distal end allows the tracking of the operating tip.

4. The instrument of claims 1 to 3, wherein the extension pole comprises a rigid arm extending from the elongated body, a platform for supporting an EM instrument receiver extending from the rigid arm, the platform allowing the EM instrument receiver to be as close as possible to the distal end without disturbing the manipulation of the instrument.

5. The instrument of claims 1 to 4, wherein each operating tip is selected from the group consisting of:
a registration probe (31) configured to designate a bone surface for registration purpose;
a drill guide configured to receive a pin for guiding a reamer or a drill bit for preparing a bone to receive a component;
a reamer configured for conforming a bone to receive an implant.

6. A system for calibrating the instrument of claims 1 to 5, comprising
- a calibration unit, comprising:
- a plate comprising a support for reproductible attachment of an EM transmitter to the plate,
- at least one receiving site arranged on the plate, the receiving site being adapted for receiving the handle of the multifunctional surgical instrument so that the distal end of the handle rests on the plate at a calibrating position, the plate and the receiving site having a known geometry;
- an EM localization system comprising: an EM transmitter arranged on the support on the plate; an EM instrument receiver (R1) attached to the extension pole;
- a processing unit configured for implementing a step of calibrating the EM instrument receiver (R1) relative to the distal end of the instrument allowing to locate the distal end of the instrument when the EM instrument receiver receives EM signals from the EM transmitter.

7. The system of claim 6, wherein the step of calibrating the EM instrument receiver comprises,
- processing EM signals received by the EM instrument receiver, while the EM transmitter emits EM signals and the instrument is rotated, in order to determine geometrical transformations of the EM transmitter relative to the EM instrument receiver;
- determining, from these geometrical transformations, the trajectory described by the EM instrument receiver (R1), said trajectory forming a circular arc (C);
- identifying the center of the circular arc within the coordinate system of the EM transmitter (T);
- determining the longitudinal axis of the instrument as the normal to the plane containing the trajectory points and passing through the center of the circular arc.
- determining the position of the distal end of the instrument by computing the intersection between the plane of the plate and the longitudinal axis of the instrument.

8. The system of claim 7, wherein the EM transmitter (T), the EM instrument receiver and the EM bone receiver are intended to be located relative to each other at a distance between 5 and 15 cm, preferably 10 cm.

9. The system of claims 6 to 8, wherein the plate is configured to be non-magnetic-field-disturbing by material and/or by design.

10. The system of claim 9, wherein a non-magnetic-field-disturbing material is plastic or titanium.
